# EUROPEAN PATENT SPECIFICATION

(11) **EP 3 243 467 B1**
(45) Date of publication and mention of the grant of the patent: **24.03.2021**
(21) Application number: 15898971.5
(22) Date of filing: 30.11.2015
(51) Int. Cl.: A61B 18/00, A61B 17/32

(54) **ULTRASOUND TREATMENT INSTRUMENT AND ULTRASOUND TREATMENT ASSEMBLY**
ULTRASCHALLBEHANDLUNGSINSTRUMENT UND ULTRASCHALLBEHANDLUNGSANORDNUNG
INSTRUMENT DE TRAITEMENT AUX ULTRASONS, ET ENSEMBLE DE TRAITEMENT AUX ULTRASONS

(30) Priority: 23.07.2015 US 201562196158 P
(43) Date of publication of application: 15.11.2017
(73) Proprietor: Olympus Corporation, Hachioji-shi, Tokyo 192-8507 (JP)
(72) Inventor: SAKAI, Masahiro, Hachioji-shi, Tokyo 192-8507 (JP)
(74) Representative: Schicker, Silvia
(86) International application number: PCT/JP2015/083589
(87) International publication number: WO 2017/013813

(56) References cited:
- EP-A1- 1 591 071
- EP-A1- 3 260 064
- WO-A1-2011/158792
- WO-A1-2012/061646
- JP-A- 2005 312 675
- JP-A- 2005 516 663
- US-A- 5 211 625

## Description

### Technical Field

The present invention relates to an ultrasonic treatment instrument and an ultrasonic treatment assembly which treat a biological tissue by use of ultrasonic vibration.

### Background Art

For example, in Jpn. Pat. Appln. KOKAI Publication No. 2003-116870, there is disclosed an ultrasonic treatment instrument to shatter and cut hard tissues such as bones. Furthermore, this treatment instrument performs suction through a suction path formed in a probe.

European Patent Application Publication No. 1 591 071 A1 discloses an ultrasonic treatment device comprising an inner sheath and a coaxial outer sheath, the inner sheath including one or more through-holes that allow communication between a distal-end suction path, realized between the distal end of a probe and the inner sheath, and a sheath-to-sheath suction path formed between the inner and outer sheaths.

U.S. Patent Application Publication No. 5 211 625 A discloses an ultrasonic treatment apparatus comprising an outer sheath having a proximal end portion coaxially extending with respect to a cover, an intermediate portion integrally connected to the distal end of the proximal end portion of the outer sheath and extending at an obtuse angle with respect to the proximal end portion, and a small-diameter distal end portion threadably engaged with the intermediate portion. The ultrasonic treatment apparatus further includes a horn and a vibration transmission member, screwed to each other at a predetermined angle. A bent portion, formed between the horn and the vibration transmission member, is covered by the distal end of the proximal end portion and the proximal end of the intermediate portion.

International Patent Application Publication No. 2012/061646 A1 discloses a surgical instrument including an outer sheath in the shape of an elongated tubular member. A plurality of holes are disposed about the cylindrical surface of the outer sheath (e.g., in a grid like pattern, in an offset grid pattern or randomly) so as to provide fluid access to the interior of the outer sheath and permitting the user to use a fluid to flush the interior of a transmission assembly to sterilize and/or resterilize the transmission assembly.

European Patent Application Publication No. 3 260 064 A1, which is a prior art document in the sense of Article 54(3) EPC, discloses an ultrasonic treatment device including a rod-like probe comprising a shaft portion having a treatment portion (i.e., an excision blade) at its distal end portion, a first hollow sheath (i.e., an inner sheath) that covers the periphery of the probe, and a second hollow sheath (i.e., an outer sheath) that covers the periphery of the first sheath. The first sheath has a first edge provided at a distal end side of the probe, and the second sheath has a second edge provided at the distal end side of the probe.

For example, in the probe of the ultrasonic treatment instrument disclosed in Jpn. Pat. Appln. KOKAI Publication No. 2003-116870, it is necessary that a treatment portion of the probe is made to access an inside of a narrow cavity such as a joint cavity. Then, the treatment portion to which ultrasonic vibration is being transmitted is brought into contact with the hard tissue of the bone or the like. Therefore, the probe is required to have suitable strength. On the other hand, to suitably maintain the strength of the probe while maintaining a size of the probe, it is necessary to decrease a transverse cross section of the suction path in the probe, but when it is so decreased, the suction path is likely to clog.

### Summary of Invention

An object of this invention is to provide an ultrasonic treatment instrument and an ultrasonic treatment assembly in which a suitable suction path is acquirable while suitably maintaining a size and strength of a probe.

The invention is an ultrasonic treatment instrument for use in arthroscopic surgery including the features of claim 1. Preferred embodiments are specified in the dependent claims. An ultrasonic treatment instrument may include: a probe having a main body that is configured to transmit ultrasonic vibration, and a treatment portion that is provided on a distal side of the main body and is configured to cut a hard tissue and/or a soft tissue by transmission of the ultrasonic vibration; and a sheath unit that includes an inner sheath covering an outer peripheral surface of the main body in the probe, and an outer sheath covering an outer peripheral surface of the inner sheath and forming a suction path between the outer peripheral surface of the inner sheath and the outer sheath, wherein a distal end of the inner sheath extends to a position closer to the treatment portion of the probe than a distal end of the outer sheath.

### Brief Description of Drawings

FIG. 1 is a schematic view showing a treatment system according to an embodiment of the invention;
FIG. 2A is a schematic view showing an ultrasonic treatment assembly of the treatment system according to the embodiment;
FIG. 2B is a schematic longitudinal cross-sectional view showing an ultrasonic treatment instrument of the ultrasonic treatment assembly of the treatment system according to the embodiment;
FIG. 3A is a schematic enlarged view of encircled parts denoted with reference signs α and β of FIG. 2B;
FIG. 3B is a schematic transverse cross-sectional view taken along the 3B-3B line of FIG. 3A;
FIG. 3C is a schematic transverse cross-sectional view taken along the 3C-3C line of FIG. 3A;
FIG. 4A is a schematic view showing a state where a cutting region and an area to be resected are observed from a rear side of a treatment portion with an arthroscope, when the cutting region of the treatment portion of the ultrasonic treatment instrument of the treatment system according to the embodiment is brought into contact with the area to be resected in a bone;
FIG. 4B is a schematic view showing a state where the cutting region and the area to be resected are laterally observed on the rear side of the treatment portion with the arthroscope, when the cutting region of the treatment portion of the ultrasonic treatment instrument of the treatment system according to the embodiment is brought into contact with the area to be resected in the bone;
FIG. 5 is a schematic enlarged view of the encircled parts denoted with the reference signs α and β of FIG. 2B in an ultrasonic treatment instrument of an ultrasonic treatment assembly of a treatment system according to a first comparative example;
FIG. 6A is a schematic enlarged view of the encircled part denoted with the reference sign α of FIG. 2B in an ultrasonic treatment instrument of an ultrasonic treatment assembly of a treatment system according to the invention;
FIG. 6B is a longitudinally cross-sectional view in which the encircled part denoted with the reference sign α of FIG. 2B is schematically enlarged in the ultrasonic treatment instrument of the ultrasonic treatment assembly of the treatment system according to the invention;
FIG. 7A is a schematic view showing an ultrasonic treatment instrument of an ultrasonic treatment assembly of a treatment system according to a second comparative example;
FIG. 7B is a schematic enlarged view of an encircled part denoted with reference sign γ of FIG. 7A;
FIG. 8A is a schematic view of the ultrasonic treatment instrument of the ultrasonic treatment assembly of the treatment system according to the second comparative example which is seen from a direction indicated by an arrow 8A in FIG. 7A;
FIG. 8B is a longitudinal cross-sectional view in which an encircled part denoted with reference sign δ of FIG. 8A is schematically enlarged;
FIG. 9A is a schematic view which shows a state where a cutout portion is formed in a distal portion of an inner sheath of an ultrasonic treatment instrument of an ultrasonic treatment assembly of a treatment system according to a third comparative example, and in which the encircled part denoted with the reference sign γ of FIG. 7A is enlarged; and
FIG. 9B is a schematic view which shows a state where a cutout portion is formed in a distal portion of an inner sheath of an ultrasonic treatment instrument of an ultrasonic treatment assembly of a treatment system according to a modification of the third comparative example, and in which the encircled part denoted with the reference sign γ of FIG. 7A is enlarged.

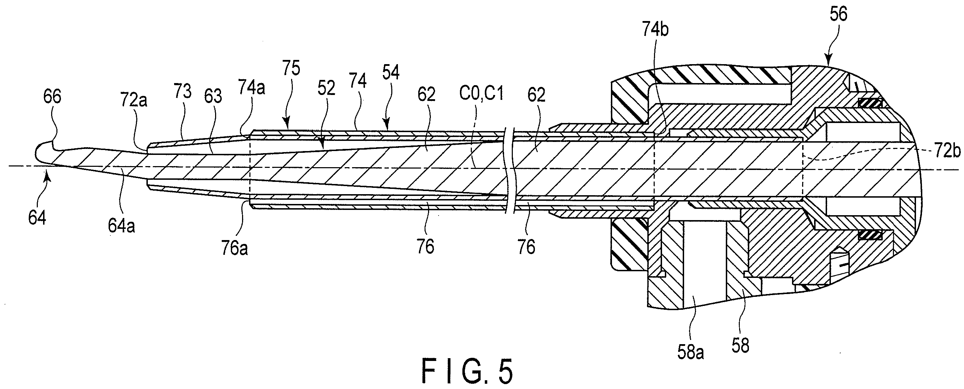

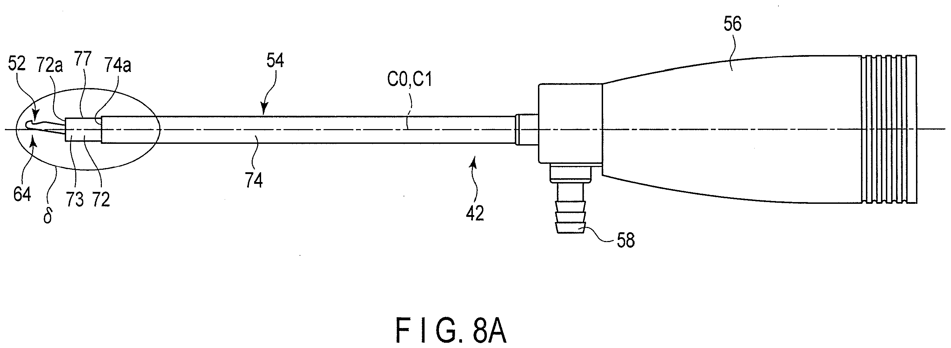

The comparative examples described below with reference to Figs. 5 and 7A to 9B only represent background that is useful for understanding the present invention, but do not form part of the same.

### Description of Embodiment and comparative examples

Hereinafter, an embodiment of this invention will be described with reference to the drawings.

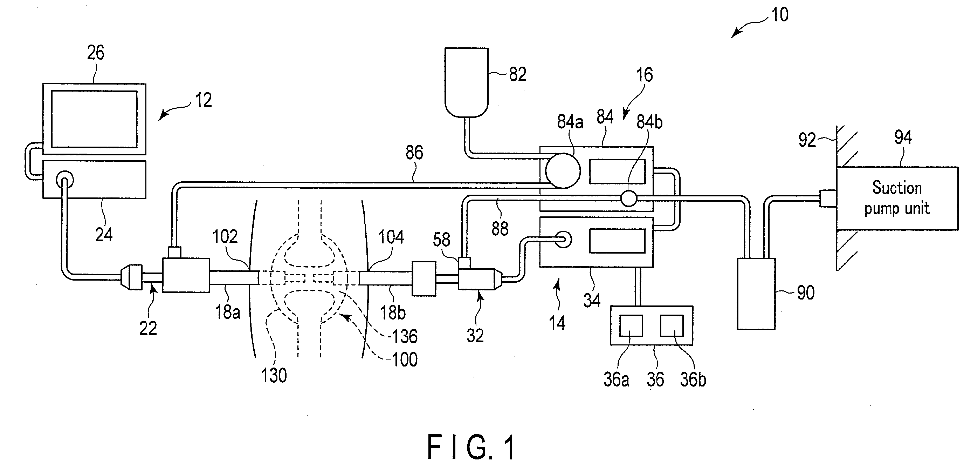

An embodiment will be described with reference to FIG. 1 to FIG. 4B.

In case of treating a joint 100, for example, a treatment system 10 shown in FIG. 1 is used. The treatment system 10 includes an arthroscope apparatus 12, a treatment apparatus 14, and a perfusion apparatus 16.

The arthroscope apparatus 12 includes an arthroscope 22 with which an inside of the joint 100 of a patient, i.e., the inside of a joint cavity 136 is observed, an arthroscope controller 24 that performs image processing on the basis of a subject image taken by the arthroscope 22, and a monitor 26 that projects a projected image generated by the image processing in the arthroscope controller 24. The arthroscope 22 is inserted into the joint cavity 136 of the joint 100 by a first cannula 18a forming an outer portal 102 that communicates between an inside of the patient's joint 100 and an outside of skin of the patient. It is to be noted that a position of the portal 102 is not uniform, but is suitably determined in accordance with patient's condition.

The treatment apparatus 14 includes an ultrasonic treatment assembly 32, a treatment instrument controller 34, and a switch 36. The ultrasonic treatment assembly 32 includes an ultrasonic treatment instrument 42, and an ultrasonic transducer unit 44 including an ultrasonic transducer 44a. As the ultrasonic transducer 44a of the ultrasonic transducer unit 44, there is used, for example, a known BLT type of transducer or the like that generates ultrasonic vibration when energy is input thereto. The ultrasonic transducer 44a of the ultrasonic transducer unit 44 can vibrate at a suitable resonance frequency, and an amplitude is changeable in a suitable range by adjustment of an amount of the energy to be input. In response to an operation of the switch 36, the treatment instrument controller 34 supplies the energy to the ultrasonic transducer unit 44, to transmit the ultrasonic vibration to a treatment portion 64 of an after-mentioned probe 52 of the ultrasonic treatment instrument 42.

The ultrasonic treatment assembly 32 is inserted into the joint cavity 136 of the joint 100 by a second cannula 18b forming an inner portal 104 that communicates between the inside of the patient's joint 100 and the outside of the skin of the patient. It is to be noted that a position of the portal 104 is not uniform but is suitably determined in accordance with the patient's condition. The switch 36, for example, in a pressed and operated state maintains a state where the ultrasonic transducer 44a of the ultrasonic transducer unit 44 is driven, and when the pressed switch is released, the driven state of the ultrasonic transducer 44a is cancelled.

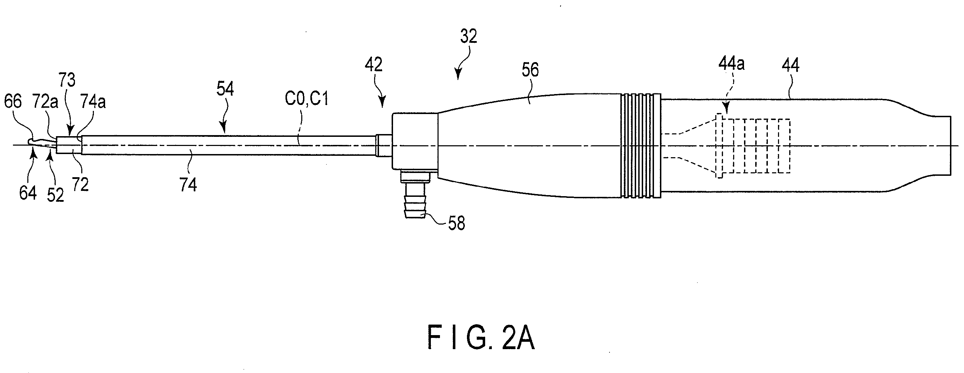

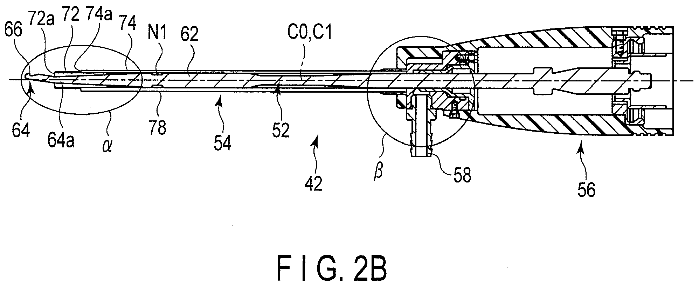

As shown in FIG. 2A to FIG. 3A, the ultrasonic treatment instrument (an ultrasonic device) 42 for use together with the arthroscope 22, i.e. , for use in arthroscopic surgery includes the probe 52, a sheath unit 54, and a handle 56 in which at least an outer peripheral surface of the handle 56 has electric insulation properties. The handle 56 is provided in a proximal portion of the sheath unit 54, and includes a connector 58 connecting an after-mentioned suction path 76 to a suction pump unit (a suction source) 94 disposed on a wall or the like in an operating room. A proximal end of the handle 56 is attachably/detachably connected to the ultrasonic transducer unit 44. Thus, the ultrasonic treatment instrument 42 is for use in a treatment in a state where the ultrasonic transducer unit 44 is attached to the instrument to form the ultrasonic treatment assembly 32.

It is to be noted that here, as shown in FIG. 1, there is described an example of use of the foot switch 36 connected to the treatment instrument controller 34 to give an instruction of inputting the energy into the ultrasonic transducer unit 44, but needless to say, it is preferable that an unshown switch (hand switch) is disposed in the handle 56. The switch 36 includes pressing portions 36a and 36b. In a case that a large vibration amplitude is generated in the transducer 44a, the pressing portion 36a is pressed, and in a case that a small vibration amplitude is generated in the transducer 44a, the pressing portion 36b is pressed. For example, the amplitude may be changed in cutting a hard tissue such as a bone and in cutting a soft tissue such as a cartilage. In other words, the amplitude may suitably be changed in accordance with a condition of a treatment target. Alternatively, the amplitude may suitably be changed in accordance with a condition of an amount of the treatment target to be cut.

In the probe 52, there is used a material such as a titanium alloy material excellent in transmission properties of the ultrasonic vibration. When the handle 56 is connected to the ultrasonic transducer unit 44, a proximal end of the probe 52 is connected to the ultrasonic transducer 44a of the ultrasonic transducer unit 44 which generates the ultrasonic vibration when the energy is input. The probe 52 includes a straight rod-like main body 62 that is configured to transmit the ultrasonic vibration, and the treatment portion 64 that is provided on a distal side of the main body 62 and is configured to cut the bone by the transmission of the ultrasonic vibration. The treatment portion 64 includes a cutting region (a cutting blade) 66 projecting in a direction different from a direction along a central axis C0 of the main body 62 of the probe 52. Furthermore, the treatment portion 64 includes a bent portion 64a bent from a distal end of the main body 62. Consequently, a position of the cutting region 66 can shift from the central axis C0 of the probe 52 in a state where the treatment portion 64 is maintained to be small.

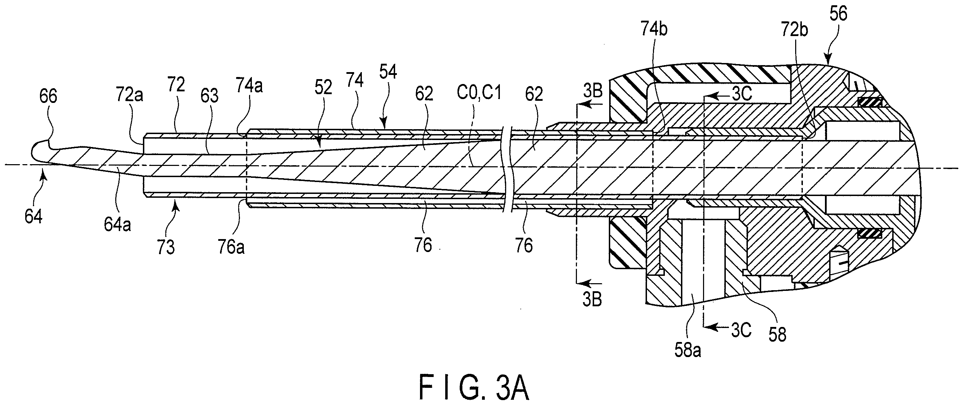

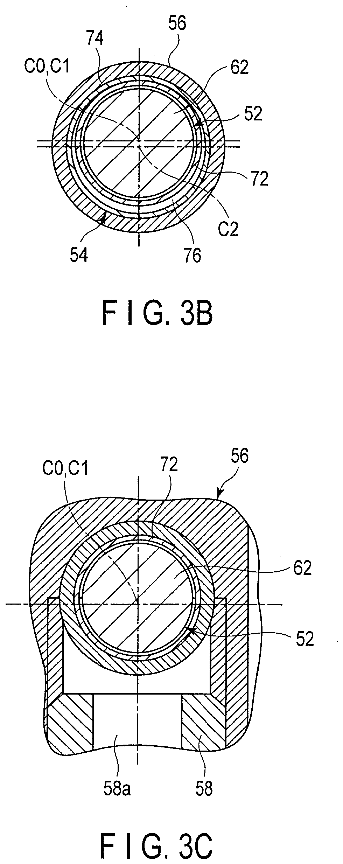

It is to be noted that the treatment portion 64 in FIG. 3A shows an example where the portion is drawn as the hook-shaped cutting region 66, but there is usable the probe 52 including the treatment portion 64 having the cutting region 66 of any type of shape such as an unshown blade or curette type.

A wavelength of the ultrasonic vibration input into the probe 52 is determined in accordance with the resonance frequency of the transducer 44a of the ultrasonic transducer unit 44. In other words, a length of the probe 52 is determined in accordance with the transducer 44a for use. Therefore, the probe 52 has the length to prescribe an antinode position of the vibration in the treatment portion 64, when the ultrasonic vibration is input from the ultrasonic transducer 44a of the ultrasonic transducer unit 44 into the proximal end of the probe 52 to transmit the ultrasonic vibration to the probe 52. It is preferable that the antinode position of the vibration matches the position of the cutting region 66. In other words, the position of the cutting region 66 is adjusted to be a position corresponding to the antinode position of the vibration. When the ultrasonic vibration is transmitted, a first node position of the vibration which is present on a proximal side of the antinode position of the vibration is prescribed between the distal end of the main body 62 of the probe 52 and a proximal end thereof.

The sheath unit 54 includes an inner sheath (a first sheath) 72 and an outer sheath (a second sheath) 74. The inner sheath 72 and the outer sheath 74 are made of a material such as a stainless alloy material having rigidity. It is preferable that a thickness of the inner sheath 72 is smaller than a thickness of the outer sheath 74. Thus, the inner sheath 72 is formed to be thin, whereby an outer diameter of the whole sheath unit 54, i.e., an outer diameter of the outer sheath 74 can be smaller.

The inner sheath 72 covers an outer peripheral surface of the main body 62 in the probe 52. A central axis C1 of the inner sheath 72 matches the central axis C0 of the main body 62 of the probe 52. A distal end 72a of the inner sheath 72 to which the ultrasonic vibration is transmitted is present on a distal side from a position denoted with reference sign N1, that is, the first node position of the vibration from the position corresponding to the antinode position of the vibration of the treatment portion 64 of the probe 52. In the main body 62 of the probe 52, an O-ring 78 is interposed between an outer peripheral surface of the position N1 that is the node position of the vibration and an inner peripheral surface of the inner sheath 72. Consequently, it is possible to prevent a liquid from entering a space between the main body 62 of the probe 52 and the inner sheath 72 on a proximal side from the O-ring 78 along the central axes C0 and C1.

The outer sheath 74 covers an outer peripheral surface of the inner sheath 72. A proximal end 72b of the inner sheath 72 and a proximal end 74b of the outer sheath 74 are fixed to the handle 56, respectively.

Furthermore, the suction path 76 is formed between the outer peripheral surface of the inner sheath 72 and an inner peripheral surface of the outer sheath 74. The proximal end 74b of the outer sheath 74 is present on the distal side from the proximal end 72b of the inner sheath 72 along the central axis C1 of the inner sheath 72, and hence the suction path 76 communicates with a communication path 58a of the connector 58 . It is to be noted that as shown in FIG. 3B, the handle 56 is fixed in a state where the central axis C1 of the inner sheath 72 shifts from a central axis C2 of the outer sheath 74. Consequently, in the suction path 76, there can be formed a portion in which a distance between the outer peripheral surface of the inner sheath 72 and the inner peripheral surface of the outer sheath 74 can be taken longer as compared with circumstances where the central axes of the inner sheath 72 and the outer sheath 74 are matched. Consequently, it is possible to suck cut pieces larger than those in the circumstances where the central axes of the inner sheath 72 and the outer sheath 74 are matched. Needless to say, it is also preferable that the central axes C1 and C2 of the inner sheath 72 and the outer sheath 74 are matched.

The distal end 72a of the inner sheath 72 extends to a position closer to the treatment portion 64 of the probe 52 than a distal end 74a of the outer sheath 74. Consequently, a region between the distal end 72a of the inner sheath 72 and the distal end 74a of the outer sheath 74, i.e. , the inner sheath 72 extends to the distal side as much as a distal portion 73 from the distal end 74a of the outer sheath 74. Therefore, a suction opening 76a of the suction path 76 is present at a position on the proximal side from the distal end 72a of the inner sheath 72 along the central axis C1.

As shown in FIG. 1, the perfusion apparatus 16 includes a bag-shaped liquid source 82 that holds perfusate such as physiological saline, a perfusion pump unit 84, a liquid feed tube 86 having one end connected to the liquid source 82, a liquid discharge tube 88, and a suction bottle 90 connected to one end of the liquid discharge tube 88. The suction bottle 90 is connected to the suction pump unit 94 attached to a wall 92 of the operating room. The perfusion pump unit 84 is capable of feeding out the perfusate from the liquid source 82 by a liquid feed pump 84a. Furthermore, the perfusion pump unit 84 is capable of opening and closing a pinch valve 84b as a liquid discharge valve, whereby it is possible to switch suction and suction stop of the perfusate in the joint cavity 136 to the suction bottle 90.

An operation of the treatment system 10 according to this embodiment will be described. The use of the treatment system does not however form part of the invention.

In case of performing an operation by use of the arthroscope 22, as shown in FIG. 2A, the ultrasonic transducer unit 44 is attached to the ultrasonic treatment instrument 42, to form the ultrasonic treatment assembly 32. Then, the connector 58 shown in FIG. 2A is connected to the liquid discharge tube 88 shown in FIG. 1, to prepare the treatment system 10 shown in FIG. 1.

As shown in FIG. 1, an operator forms the first portal 102, and disposes the first cannula 18a in the first portal 102 as required. The operator disposes a distal end of the arthroscope 22 in the joint cavity 136 of the joint 100 through the first cannula 18a. Here, when the perfusion apparatus 16 is connectable to the arthroscope 22, the first cannula 18a is not necessarily required. The operator forms the second portal 104 and disposes the second cannula 18b in the second portal 104. It is to be noted that here, an example of use of the second cannula 18b is described, but the second cannula 18b is not necessarily required.

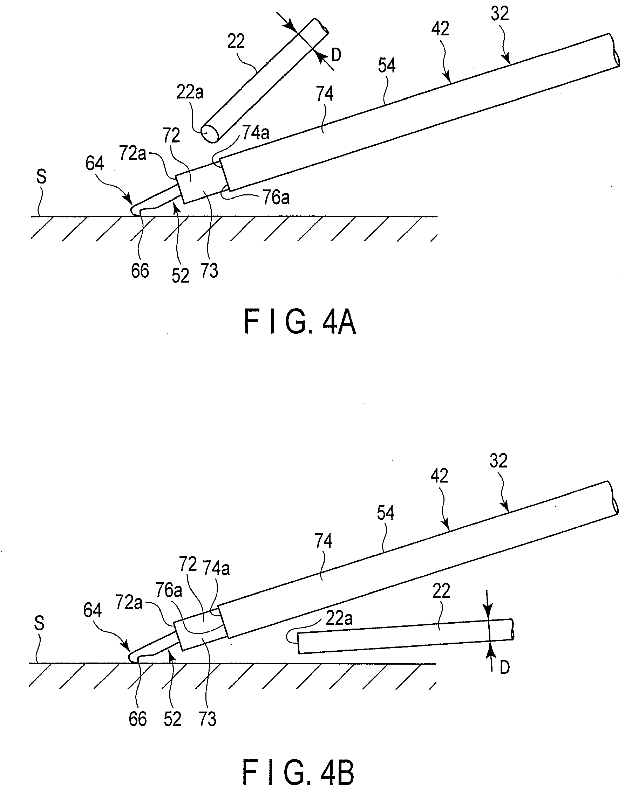

As to the ultrasonic treatment assembly 32 that performs the cutting of the bone, the treatment portion 64 of the probe 52 and the sheath unit 54 are inserted together with the arthroscope 22 into a narrow joint capsule 130. Then, the operator brings the cutting region 66 of the treatment portion 64 into contact with an area (a treatment target) S to be resected in the bone (see FIG. 4A and FIG. 4B) on the basis of the projected image observed with the arthroscope 22. In a state where the cutting region 66 is inserted in a viewing field of the arthroscope 22, the operator operates the switch 36 to supply the energy to the ultrasonic transducer unit 44 while operating the suction pump unit 94. Ultrasonic vibration is generated in the ultrasonic transducer 44a of the ultrasonic transducer unit 44, and the ultrasonic vibration is input into the probe 52. Thus, the ultrasonic vibration is transmitted to the cutting region 66 of the treatment portion 64, whereby a treatment of resecting the area S to be resected in the bone can be performed with the cutting region 66. By this treatment, a hard tissue such as a bone spur can be removed. The operator removes, by suction, bubbles or cut pieces generated during the treatment by use of the ultrasonic treatment instrument 42, and acquires the viewing field of the arthroscope 22. Parts of the cut pieces of the bone are fine particles. Consequently, the resected and cut pieces are sucked from the suction opening 76a between the outer peripheral surface of the inner sheath 72 and the inner peripheral surface of the outer sheath 74.

It is to be noted that the cut pieces that cannot enter the suction opening 76a are floated in the joint cavity and collected by using forceps or the like as required.

When the treatment portion 64 of the probe 52 is disposed to face a distal end 22a of the arthroscope 22 as shown in FIG. 1, it is possible to easily observe the cutting region 66 of the treatment portion 64 and the area S to be resected in the bone with the arthroscope 22. On the other hand, as shown in FIG. 4A and FIG. 4B, the treatment is occasionally performed while observing the treatment portion 64 of the probe 52 from a rear side with the arthroscope 22. At this time, when it is supposed that the distal end 74a of the outer sheath 74 extends to the same position as the distal end 72a of the inner sheath 72, it can easily be considered that the distal end 74a of the outer sheath 74 which has the outer diameter larger than an outer diameter of the inner sheath 72 would obstruct the observation with the arthroscope 22. As shown in FIG. 3A, in this embodiment, the distal end 72a of the inner sheath 72 extends from the distal end 74a of the outer sheath 74 on the distal side along the central axis C1, to project the cutting region 66 of the treatment portion 64 of the probe 52 from the distal end 72a of the inner sheath 72. In other words, the distal end 72a of the inner sheath 72 extends farther than the distal end 74a of the outer sheath 74, that is, the suction opening 76a, to cover the bent portion 64a of the treatment portion 64 of the probe 52. Again in other words, the suction opening 76a is formed at the position on the proximal side along the central axes C1 and C2 to the distal end 72a of the inner sheath 72. The outer diameter of the inner sheath 72 is smaller than that of the outer sheath 74, and hence when the distal end 72a of the inner sheath 72 extends to the distal side from the distal end 74a of the outer sheath 74, it is easy to confirm the cutting region 66 of the treatment portion 64 and the area S to be resected in the bone from the rear side with the arthroscope 22. In other words, according to this embodiment, as compared with circumstances where the distal end 74a of the outer sheath 74 extends to the same position as the distal end 72a of the inner sheath 72, it is easier to observe the cutting region 66 of the treatment portion 64 of the probe 52 with the arthroscope 22, because the presence of the outer sheath 74 having the outer diameter larger than that of the inner sheath 72 is eliminated in the distal portion 73 of the inner sheath 72 (a region of the inner sheath 72 between the distal end 72a of the inner sheath 72 and the distal end 74a of the outer sheath 74).

Furthermore, the bent portion 64a of the treatment portion 64 of the probe 52 is covered with the distal portion 73 of the inner sheath 72. In particular, a proximal portion of the bent portion 64a of the treatment portion 64 of the probe 52 is covered with the distal portion 73 of the inner sheath 72. Consequently, when the distal end of the arthroscope 22 is brought close to the cutting region 66 from the rear side of the treatment portion 64 to insert the cutting region into the viewing field, the bent portion 64a of the treatment portion 64 is prevented from being brought into contact with the arthroscope 22, by the distal portion 73 of the inner sheath 72. It is to be noted that when the distal end of the arthroscope 22 is brought close to the cutting region 66 from the rear side of the treatment portion 64 to insert the region into the viewing field, needless to say, the bent portion 64a of the treatment portion 64 is occasionally prevented from being brought into contact with the arthroscope 22, by the outer peripheral surface of the outer sheath 74 depending on a relative position between the sheath unit 54 and the arthroscope 22.

It is to be noted that here, the example of the treatment of cutting a hard tissue such as the bone is described, but also in case of performing a treatment of removing a part of a comparatively soft tissue such as the cartilage, the same treatment assembly 32 is usable. Furthermore, also in a case of treating the hard tissue or a case of treating the soft tissue, it is preferable that the amplitude of the transducer 44a is suitably selected to proceed with the treatment.

As described above, the ultrasonic treatment assembly 32 and the ultrasonic treatment instrument 42 according to this embodiment can be considered as follows.

In this structure, the distal end 72a of the inner sheath 72 extends farther than the distal end 74a of the outer sheath 74 (the suction opening 76a), to cover the bent portion 64a of the treatment portion 64 of the probe 52, whereby the distal portion 73 is formed. Moreover, the bent portion 64a of the probe 52 is covered with the distal portion 73 of the inner sheath 72. Therefore, the bent portion 64a of the probe 52 can be protected from the contact with the arthroscope 22, by the distal portion 73 of the inner sheath 72. Consequently, a distal portion 63 of the main body 62 of the probe 52 (a region of the bent portion 64a on the proximal side) which is shown in FIG. 3A is protectable by the inner sheath 72.

In addition, the distal end 72a of the inner sheath 72 extends farther than the distal end 74a of the outer sheath 74 (the suction opening 76a) . Thus, due to the structure where the inner sheath 72 having the small outer diameter extends farther than the outer sheath 74, it is possible to improve visibility of the cutting region 66 with the arthroscope 22. Additionally, due to the structure where the inner sheath 72 having the small outer diameter extends farther than the outer sheath 74, it is possible to improve visibility of the area S to be resected in the bone.

Therefore, due to the structure where the inner sheath 72 having the small diameter extends to cover the bent portion 64a of the treatment portion 64 of the probe 52 on the distal side from the suction opening 76a, it is possible to acquire easiness of the treatment in the cutting region 66 of the treatment portion 64 while preventing the arthroscope 22 from coming in contact with the bent portion 64a of the treatment portion 64 of the probe 52.

Here, it is not necessary to form a suction path in the probe 52 itself. Therefore, the size and strength of the probe 52 can be maintained in a suitable state. At this time, the suction path 76 is formed between the outer peripheral surface of the inner sheath 72 and the inner peripheral surface of the outer sheath 74. Consequently, the suitable suction path 76 can be acquired. In this embodiment, the central axis C1 of the inner sheath 72 shifts slightly from the central axis C2 of the outer sheath 74, whereby a broad portion and a narrow portion can be formed in the suction path 76, and it is possible to inhibit the suction path from being clogged with the comparatively large cut pieces and the like.

FIG. 5 shows an ultrasonic treatment instrument of an ultrasonic treatment assembly according to a first comparative example. In this comparative example, the same members or members having the same functions as in the members described in the embodiment are denoted with the same reference signs, and detailed description is omitted.

In the embodiment, as shown in FIG. 2A to FIG. 3A, the example where the inner sheath 72 has the straight state has been described. In this comparative example, as shown in FIG. 5, a distal portion 73 of an inner sheath 72 is formed so that its outer diameter decreases toward a distal side. In this case, it is preferable that not only an inner diameter but also the outer diameter of the distal portion 73 of the inner sheath 72 decrease toward the distal side. It is preferable that the diameter of the distal portion 73 of the inner sheath 72 decreases from its proximal side toward the distal side. It is preferable that the distal portion 73 of the inner sheath 72 is formed into, for example, a tapered shape so that its diameter decreases toward the distal side.

In case of FIG. 5, the distal portion 73 of the inner sheath 72 is formed into a state where its diameter decreases toward the distal side, whereby when a cutting region 66 is observed with an arthroscope 22, the observation of the cutting region 66 can be prevented from being obstructed by the distal portion 73 of the inner sheath 72 as compared with the embodiment mentioned above. Furthermore, a distal portion 63 of a main body 62 of a probe 52 (a region of a bent portion 64a on the proximal side) can be protected by the presence of the distal portion 73 of the inner sheath 72.

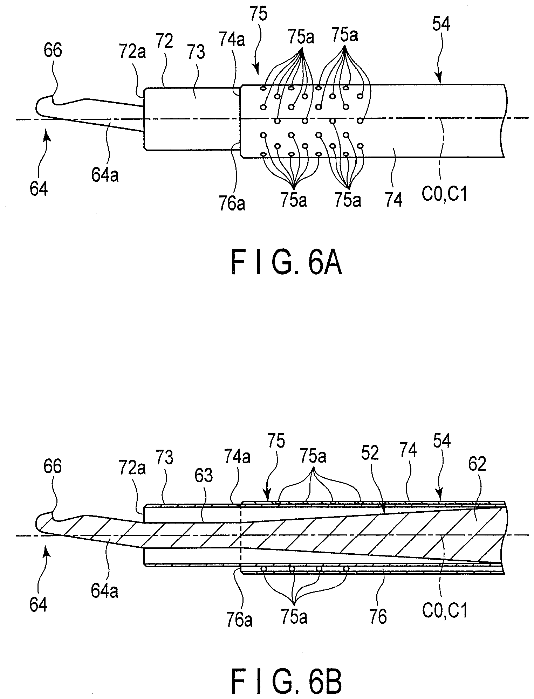

According to the invention, as shown in Figs. 6A-6B, a distal portion (a region having a suitable length (e.g., from several millimeters to several tens of millimeters) from a distal end 74a of the outer sheath 74 along a central axis C2 toward a proximal side) 75 of the outer sheath 74 has through-holes 75a which communicate between an outer peripheral surface of the outer sheath 74 and an inner peripheral surface thereof. In other words, the through-holes 75a communicate between the outer peripheral surface of the outer sheath 74 and a suction path 76. A hole diameter of the through-holes 75a is suitably settable. For example, the hole diameter may be larger or smaller than a width of a suction opening 76a in a radial direction which is prescribed by an outer peripheral surface of an inner sheath 72 and an inner peripheral surface of the distal end 74a of the outer sheath 74. Needless to say, the hole diameter of the through-holes 75a is set to a size so that when an arthroscope 22 comes in contact with the outer peripheral surface of the outer sheath 74, the arthroscope does not contact the outer peripheral surface of the inner sheath 72. As one example, the hole diameter of the through-holes 75a may be of the same degree as or smaller than a size of presumed cut pieces of a bone.

An operation of a treatment system 10 according to the embodiment will be described. The use of the treatment system does not however form part of the invention.

For example, when a membranous biological tissue such as a synovial membrane (not shown) in a joint cavity 136 covers the suction opening 76a, the suction opening 76a is clogged. In this case, as described above, the suction path 76 is cut off. In the embodiment, the through-holes 75a have a suitable diameter, and hence the through-holes 75a are used as suction openings together with the suction opening 76a. Consequently, even when the suction opening 76a is clogged, particles of cut pieces can continue to be sucked through the through-holes 75a and the suction path 76. In consequence, due to the through-holes 75a, the suction opening can be prevented from being clogged with the cut pieces, and it is easy to always acquire a viewing field by the arthroscope 22.

As described above, an ultrasonic treatment assembly 32 and an ultrasonic treatment instrument 42 according to the embodiment can be considered as follows.

The cut pieces generated by a cutting treatment with a cutting region 66 of a treatment portion 64 are occasionally stuck on the suction opening 76a. In the distal portion 75 of the outer sheath 74, the optional number of the through-holes 75a each having an optional diameter are formed in an optional direction, whereby even when the suction opening 76a is closed, it is possible to prevent the suction through the suction path 76 from being stopped because the suction is performed from the through-holes 75a. In consequence, it is possible to always acquire the observation viewing field of the arthroscope 22.

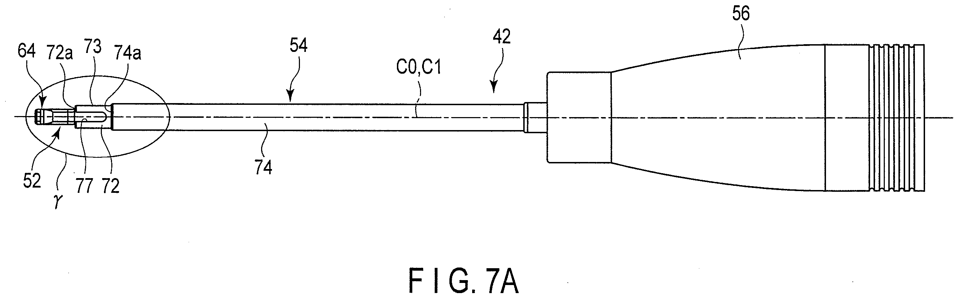

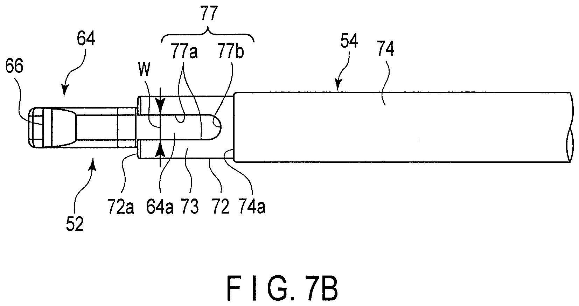

Next, a second comparative example will be described with reference to FIG. 7A to FIG. 8B.

According to the present comparative example, an inner sheath 72 includes a cutout portion 77 extending from a distal end 72a toward a predetermined position on a proximal side. The cutout portion 77 is formed especially in a distal portion 73 of the inner sheath 72. The cutout portion 77 is straightly formed in parallel along a central axis C1. A length of the cutout portion 77 in a direction along the central axis C1 of the inner sheath 72 is substantially equal to a length of the distal portion 73 in the central axis C1. The cutout portion 77 includes a pair of edge portions 77a straightly formed in parallel along the central axis C1 and facing each other, and a semicircular bottom portion 77b provided at proximal ends of the edge portions 77a. The proximal ends of the pair of edge portions 77a are smoothly continuous with the bottom portion 77b. The cutout portion 77 is formed on a projecting side of the distal portion 73 of the inner sheath 72 on which a cutting region 66 of a treatment portion 64 of a probe 52 is present.

A width W of the cutout portion 77 between the pair of edge portions 77a which is perpendicular to the central axis C1 of the inner sheath 72 is smaller than a diameter D (see FIG. 4A and FIG. 4B) of a minimum region of a distal portion of an arthroscope 22. Furthermore, a suitable clearance is formed between an inner peripheral surface of the distal portion 73 of the inner sheath 72 and the bent portion 64a of the treatment portion 64 of the probe 52.

An operation of a treatment system 10 according to this comparative example will be described.

The cutout portion (a groove) 77 having the width W smaller than the diameter D of the arthroscope 22 is formed in the distal portion 73 of the inner sheath 72. Consequently, as shown in FIG. 4B, when an area S to be resected in a bone is observed with the arthroscope 22 and the cutting region 66 of the treatment portion 64 is observed therewith, the area S and the cutting region 66 are both observed through the cutout portion 77 of the inner sheath 72.

Furthermore, even when the arthroscope 22 contacts the cutout portion 77, the arthroscope 22 is prevented from coming in contact with the bent portion 64a of the treatment portion 64 of the probe 52. In particular, when the cutting region 66 of the treatment portion 64 is brought into contact with the area S to be resected in the bone, the bent portion 64a is elastically deformed in a direction away from the cutout portion 77. Consequently, a distance between the bent portion 64a of the treatment portion 64 of the probe 52 and the cutout portion 77 of the distal portion 73 of the inner sheath 72 increases. Therefore, the arthroscope 22 is prevented from coming in contact with the probe 52 to which ultrasonic vibration is being transmitted.

As described above, an ultrasonic treatment assembly 32 and an ultrasonic treatment instrument 42 according to this comparative example can be considered as follows.

The inner sheath 72 extends as much as the distal portion 73 from a distal end 74a of an outer sheath 74 on a distal side.
In this comparative example, the cutout portion 77 is formed in the distal portion 73 of the inner sheath 72, whereby it is easier to insert the cutting region 66 of the treatment portion 64 into a viewing field of the arthroscope 22 through the cutout portion 77 of the inner sheath 72, as compared with circumstances where the cutout portion 77 is not present. Furthermore, it is possible to observe the area S to be resected in the bone while observing the cutting region 66 of the treatment portion 64 through the cutout portion 77 of the inner sheath 72 with the arthroscope 22. In consequence, it is possible to acquire the observation viewing field of the arthroscope 22.

Furthermore, the width W of the cutout portion 77 of the inner sheath 72 is smaller than the diameter D of the arthroscope 22. In consequence, the arthroscope 22 can be prevented from coming in contact with the treatment portion 64 of the probe 52.

Thus, the width W of the cutout portion 77 formed in the inner sheath 72 is smaller than the diameter D of the arthroscope 22, whereby it can be easier to insert the cutting region 66 of the treatment portion 64 into the viewing field of the arthroscope 22 while preventing the arthroscope 22 from coming in contact with the treatment portion 64 of the probe 52.

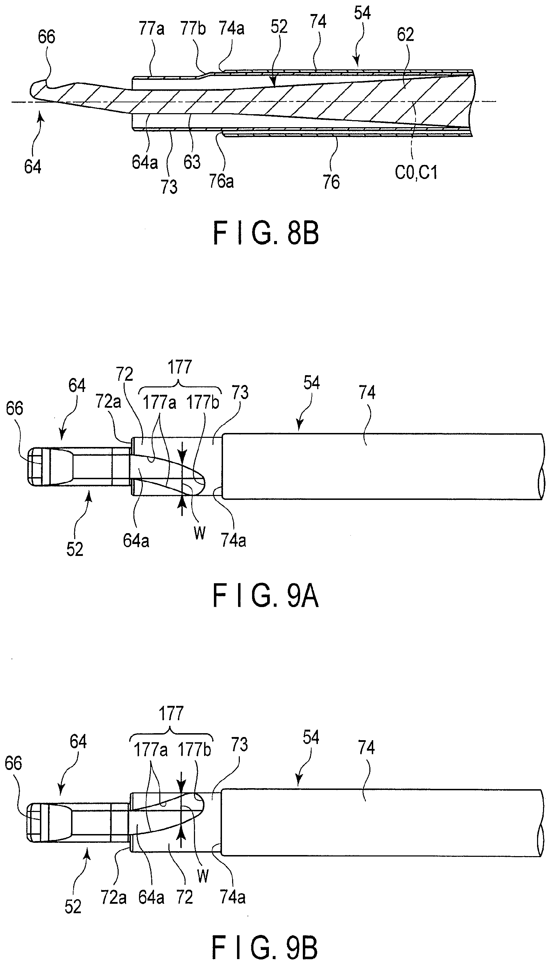

Next, a third comparative example will be described with reference to FIG. 9A and FIG. 9B.

As shown in FIG. 9A, a cutout portion 177 is formed in a distal portion 73 of an inner sheath 72. The cutout portion 177 includes a pair of edge portions 177a and a bottom portion 177b. The pair of edge portions 177a are not formed in parallel with a central axis C1 of the inner sheath 72, but are obliquely formed. In other words, the cutout portion 177 is formed to tilt to the central axis C1 of the inner sheath 72. It is preferable that the pair of edge portions 177a are parallel to each other.

When a treatment of cutting a bone of a treatment target is performed, a cutting region 66 of a treatment portion 64 is observed obliquely from a rear side with an arthroscope 22 in most cases. Consequently, in a state where a positional relation between the arthroscope 22 and the treatment portion 64 is determined, the edge portions 177a of the cutout portion 177 may have a straight state, but when the edge portions obliquely extend, it can be easy to observe the cutting region 66 of the treatment portion 64 with the arthroscope 22. In particular, when the arthroscope 22 and a treatment instrument 42 have such a state as shown in FIG. 4A, that is, when the arthroscope 22 is disposed on a right rear side of the treatment instrument 42, and when the cutout portion 177 having such a shape as shown in FIG. 9A is formed, it can be easy to observe the treating region 66.

An extending direction of the cutout portion 177 shown in FIG. 9A may be a direction shown in FIG. 9B. This can further contribute to easiness of the observation of the treatment portion 64, when a positional relation between portals 102 and 104 (see FIG. 1) is determined. In particular, when the arthroscope 22 is disposed on a left rear side of the treatment instrument 42 conversely to the state of the arthroscope 22 and the treatment instrument 42 shown in FIG. 4A and when the cutout portion 177 having such a shape as shown in FIG. 9B is formed, it can be easy to observe the cutting region 66.

Here, there is described the example where the pair of edge portions 177a of the cutout portion 177 are parallel to each other as shown in FIG. 9A and FIG. 9B. Alternatively, it is also preferable that a cutout width between the pair of edge portions 177a decreases toward a distal end 72a of the inner sheath 72.

### Reference Signs List

10...treatment system, 12...arthroscope apparatus, 14...treatment apparatus, 16...perfusion apparatus, 22...arthroscope, 32...ultrasonic treatment assembly, 36...switch(footswitch), 42...ultrasonic treatment instrument, 44...ultrasonic transducer unit, 44a...ultrasonic transducer, 52...probe, 54...sheath unit, 56...handle, 62...main body, 63...distal portion, 64...treatment portion, 64a····bent portion, 66···cutting region, 72···inner sheath, 73···distal portion, 74···outer sheath, C0,C1,C2 ··· central axis.

## Claims

1. An ultrasonic treatment instrument (42) for use in arthroscopic surgery, comprising:
a probe (52) having:
a main body (62) that is configured to transmit ultrasonic vibration, and
a treatment portion (64) that is provided on a distal side of the main body (62) and is configured to cut a hard tissue and/or a soft tissue by transmission of the ultrasonic vibration,
the main body (62) prescribing a central axis (C0),
the treatment portion (64) including:
a bent portion (64a) bent from a distal end of the main body (62) that is continuous with the main body (62) and is bent from a distal end of the main body (62), and
a cutting region (66) provided on a distal side of the bent portion (64a) and formed at a position shifted from the central axis (C0) due to the bent portion (64a); and
a sheath unit (54) that includes:
an inner sheath (72) including a distal portion (73) which covers the main body (62) present in a region on a proximal side of the bent portion (64a) in the probe (52) and covers a region bent from the distal end of the main body (62) in a proximal portion of the bent portion (64a), and
an outer sheath (74) covering an outer peripheral surface of the inner sheath (72) and forming a suction path (76) between the outer peripheral surface of the inner sheath (72) and the outer sheath (74),
wherein
a distal end (72a) of the inner sheath (72) extends to a position distally closer to the cutting region (66) of the treatment portion (64) than a distal end (74a) of the outer sheath (74), and
through-holes (75a) communicating between an outer peripheral surface of the outer sheath (74) and an inner peripheral surface of the outer sheath (74) are formed in a distal portion of the outer sheath (74).

2. The ultrasonic treatment instrument (42) according to claim 1, wherein a central axis (C2) of the outer sheath (74) is shifted from a central axis (C1) of the inner sheath (72).

3. The ultrasonic treatment (42) instrument according to claim 1, wherein a thickness of the inner sheath (72) is smaller than a thickness of the outer sheath (74).

4. The ultrasonic treatment instrument (42) according to claim 1, comprising:
a connector (58) that is provided on a proximal portion of the sheath unit (54) and connecting the suction path (76) to a suction source (94).

5. The ultrasonic treatment instrument (42) according to claim 1, wherein
the probe (52) has a length to include an antinode position of the vibration in the treatment portion (64), when the ultrasonic vibration is transmitted thereto, and
the distal end (72a) of the inner sheath (72) is present between the antinode position of the vibration in the treatment portion (64) and a position corresponding to a first node position (N1) of the vibration that is separated from the antinode position of the vibration, in the probe (52).

6. The ultrasonic treatment instrument (42) according to claim 1, wherein
a central axis (C0) of the main body (62) of the probe (52) matches a central axis (C1) of the inner sheath (72), and
the central axis (C1) of the inner sheath (72) does not match a central axis (C2) of the outer sheath (74).

7. An ultrasonic treatment assembly (32) comprising:
the ultrasonic treatment instrument (42) according to claim 1; and
an ultrasonic transducer unit (44) detachably attached to the ultrasonic treatment instrument (42).

## Patentansprüche

1. Ultraschall-Behandlungsinstrument (42) zur Verwendung in der arthroskopischen Chirurgie, das umfasst:
eine Sonde (52) mit:
einem Hauptkörper (62), der so konfiguriert ist, dass er Ultraschallschwingungen überträgt, und
einem Behandlungsabschnitt (64), der an einer distalen Seite des Hauptkörpers (62) vorgesehen ist und so konfiguriert ist, dass er ein hartes Gewebe und/oder ein weiches Gewebe durch Übertragung der Ultraschallschwingung schneidet, wobei
der Hauptkörper (62) eine zentrale Achse (C0) vorgibt,
der Behandlungsabschnitt (64) umfasst:
einen von einem distalen Ende des Hauptkörpers (62) aus gebogenen gebogenen Abschnitt (64a), der mit dem Hauptkörper (62) zusammenhängt von einem distalen Ende des Hauptkörpers (62) aus gebogen ist, und
einen Schneidbereich (66), der an einer distalen Seite des gebogenen Abschnitts (64a) vorgesehen ist und an einer Position ausgebildet ist, die aufgrund des gebogenen Abschnitts (64a) von der zentralen Achse (C0) versetzt ist; und
eine Hülleneinheit (54), die umfasst:
eine innere Hülle (72) mit einem distalen Abschnitt (73), der den Hauptkörper (62) bedeckt, der in einem Bereich auf einer proximalen Seite des gebogenen Abschnitts (64a) in der Sonde (52) vorhanden ist, und einen von dem distalen Ende des Hauptkörpers (62) aus gebogenen Bereich in einem proximalen Abschnitt des gebogenen Abschnitts (64a) bedeckt, und
eine äußere Hülle (74), die eine Außenumfangsfläche der innere Hülle (72) bedeckt und einen Saugpfad (76) zwischen der Außenumfangsfläche der inneren Hülle (72) und der äußeren Hülle (74) bildet,
wobei
ein distales Ende (72a) der inneren Hülle (72) sich zu einer Position erstreckt, die distal näher an dem Schneidbereich (66) des Behandlungsabschnitts (64) liegt als ein distales Ende (74a) der äußeren Hülle (74), und
Durchgangslöcher (75a), die zwischen einer Außenumfangsfläche der äußeren Hülle (74) und einer Innenumfangsfläche der äußeren Hülle (74) kommunizieren, in einem distalen Abschnitt der äußeren Hülle (74) ausgebildet sind.

2. Ultraschall-Behandlungsinstrument (42) nach Anspruch 1, wobei eine zentrale Achse (C2) der äußeren Hülle (74) gegenüber einer zentralen Achse (C1) der inneren Hülle (72) verschoben ist.

3. Ultraschall-Behandlungsinstrument (42) nach Anspruch 1, wobei eine Dicke der inneren Hülle (72) kleiner ist als eine Dicke der äußeren Hülle (74).

4. Ultraschall-Behandlungsinstrument (42) nach Anspruch 1, das umfasst:
ein Verbindungsstück (58), das an einem proximalen Abschnitt der Hülleneinheit (5 4) vorgesehen ist und den Saugpfad (76) mit einer Saugquelle (94) verbindet.

5. Ultraschall-Behandlungsinstrument (42) nach Anspruch 1, wobei
die Sonde (52) eine Länge hat, die eine Antinodenposition der Schwingung in dem Behandlungsabschnitt (64) umfasst, wenn die Ultraschallschwingung daran übertragen wird, und
das distale Ende (72a) der inneren Hülle (72) sich zwischen der Antinodenposition der Schwingung in dem Behandlungsabschnitt (64) und einer Position befindet, die einer ersten Nodenposition (N1) der Schwingung in der Sonde (52) entspricht, die von der Antinodenposition der Schwingung getrennt ist.

6. Ultraschall-Behandlungsinstrument (42) nach Anspruch 1, wobei
eine zentrale Achse (C0) des Hauptkörpers (62) der Sonde (52) mit einer zentralen Achse (C1) der inneren Hülle (72) übereinstimmt, und
die zentrale Achse (C1) der inneren Hülle (72) nicht mit einer zentralen Achse (C2) der äußeren Hülle (74) übereinstimmt.

7. Ultraschallbehandlungsanordnung (32), die umfasst:
das Ultraschall-Behandlungsinstrument (42) nach Anspruch 1; und
eine Ultraschall-Wandlereinheit (44), die lösbar an dem Ultraschall-Behandlungsinstrument (42) angebracht ist.

## Revendications

1. Instrument (42) de traitement par ultrasons pour une utilisation en chirurgie arthroscopique, comprenant :
une sonde (52) ayant :
un corps principal (62) qui est configuré pour transmettre une vibration ultrasonore, et
une partie (64) de traitement qui est prévue sur un côté distal du corps principal (62) et est configurée pour couper un tissu dur et/ou un tissu mou par transmission de la vibration ultrasonore,
le corps principal (62) déterminant un axe central (C0),
la partie (64) de traitement incluant :
une partie courbée (64a) courbée depuis une extrémité distale du corps principal (62) qui est continue avec le corps principal (62) et est courbée depuis une extrémité distale du corps principal (62), et
une région coupante (66) prévue sur un côté distal de la partie courbée (64a) et formée en une position décalée par rapport à l'axe central (C0) du fait de la partie courbée (64a) ; et
une unité (54) de gaine qui inclut :
une gaine intérieure (72) incluant une partie distale (73) qui recouvre le corps principal (62) présent dans une région sur un côté proximal de la partie courbée (64a) dans la sonde (52) et recouvre une région courbée depuis l'extrémité distale du corps principal (62) dans une partie proximale de la partie courbée (64a), et
une gaine extérieure (74) recouvrant une surface périphérique extérieure de la gaine intérieure (72) et formant un chemin (76) d'aspiration entre la surface périphérique extérieure de la gaine
intérieure (72) et la gaine extérieure (74),
dans lequel
une extrémité distale (72a) de la gaine intérieure (72) s'étend jusqu'à une position distalement plus proche de la région coupante (66) de la partie (64) de traitement qu'une extrémité distale (74a) de la gaine extérieure (74), et
des trous traversants (75a) communiquant entre une surface périphérique extérieure de la gaine extérieure (74) et une surface périphérique intérieure de la gaine extérieure (74) sont formés dans une partie distale de la gaine extérieure (74).

2. Instrument (42) de traitement par ultrasons selon la revendication 1, dans lequel un axe central (C2) de la gaine extérieure (74) est décalé par rapport à un axe central (C1) de la gaine intérieure (72).

3. Instrument (42) de traitement par ultrasons selon la revendication 1, dans lequel une épaisseur de la gaine intérieure (72) est plus petite qu'une épaisseur de la gaine extérieure (74).

4. Instrument (42) de traitement par ultrasons selon la revendication 1, comprenant :
un connecteur (58) qui est prévu sur une partie proximale de l'unité (54) de gaine et connectant le chemin (76) d'aspiration à une source (94) d'aspiration.

5. Instrument (42) de traitement par ultrasons selon la revendication 1, dans lequel
la sonde (52) a une longueur pour inclure une position d'antinœud de la vibration dans la partie (64) de traitement, lorsque la vibration ultrasonore est transmise à celle-ci, et
l'extrémité distale (72a) de la gaine intérieure (72) est présente entre la position d'antinœud de la vibration dans la partie (64) de traitement et une position correspondant à une première position de nœud (N1) de la vibration qui est séparée de la position d'antinœud de la vibration, dans la sonde (52).

6. Instrument (42) de traitement par ultrasons selon la revendication 1, dans lequel
un axe central (C0) du corps principal (62) de la sonde (52) correspond à un axe central (C1) de la gaine intérieure (72), et
l'axe central (C1) de la gaine intérieure (72) ne correspond pas à un axe central (C2) de la gaine extérieure (74).

7. Ensemble (32) de traitement par ultrasons comprenant :
l'instrument (42) de traitement par ultrasons selon la revendication 1 ; et une unité (44) de transducteur d'ultrasons fixée de façon détachable à l'instrument (42) de traitement par ultrasons.
